# EUROPÄISCHE PATENTSCHRIFT

(11) **EP 2 484 659 B1**
(45) Veröffentlichungstag und Bekanntmachung des Hinweises auf die Patenterteilung: **19.03.2014**
(21) Anmeldenummer: 12150709.9
(22) Anmeldetag: 11.01.2012
(51) Int. Cl.: C07C 209/16, C07C 211/09, C07C 211/12

(54) **Herstellung von in ihrer Hauptkette linearen primären Diaminen für Polyamidsynthesen**
Production of linear main chain primary diamines for polyamide syntheses
Fabrication de diamines primaires linéaires dans leur chaîne principale pour synthèses polyamidiques

(30) Priorität: 03.02.2011 DE 102011003595
(43) Veröffentlichungstag der Anmeldung: 08.08.2012
(73) Patentinhaber: Evonik Degussa GmbH, 45128 Essen (DE)
(72) Erfinder: Baumann, Franz-Erich, Dr., 48249 Dülmen (DE); Martin, Andreas, Dr., 12524 Berlin (DE); Ullrich, Matthias, Dr., 34132 Kassel (DE); Roos, Martin, Dr., 45721 Haltern am See (DE); Hannen, Peter, Dr., 45701 Herten (DE); Petrat, Frank-Martin, Dr., 48151 Münster (DE); Häger, Harald, Dr., 59348 Lüdinghausen (DE); Köckritz, Angela, Dr., 12437 Berlin (DE); Walther, Guido, 18059 Rostock (DE); Deutsch, Jens, Dr., 15834 Rangsdorf (DE)

(56) Entgegenhaltungen:
- WO-A1-2010/018570
- GB-A- 1 195 287

## Beschreibung

Die Erfindung betrifft die katalytische Umwandlung von in ihrer Hauptkette linearen Diolen mit Kohlenstoffzahlen von C4 bis C31 zu den korrespondierenden Diaminen. Die Reaktion erfolgt in flüssiger oder überkritischer Phase unter Verwendung homogener Ruthenium-haltiger Komplexe als Katalysatoren. Der Vorteil der Erfindung ist die Erzeugung primärer Diamine in einer solchen Selektivität und Reinheit, dass sie für Polyamidsynthesen geeignet sind.

Die in dem erfindungsgemäßen Verfahren verwendeten Diole werden mit Ammoniak in einem Schritt ohne zusätzliche Anwesenheit molekularen Wasserstoffs direkt zu den korrespondierenden Diaminen umgesetzt. Der dazu benötigte Ruthenium-haltige Katalysator enthält vorzugsweise neben dem Aktivmetall einen oder mehrere koordinierend wirkende Liganden aus der Gruppe der Phosphor(III)-Verbindungen bzw. Liganden, die sowohl P(III)-haltige Gruppen als auch weitere koordinierende Zentren für das Aktivmetall enthalten.

Für die Umwandlung von Alkoholen mit Ammoniak zu primären Aminen sind in Wissenschaft und Technik eine Reihe von Verfahren in flüssiger, gasförmiger oder überkritischer Phase beschrieben worden (GB 1 195 287).

Die Herausforderung für diese Verfahren besteht dabei im Erreichen hoher Selektivitäten zum primären Amin. Da Alkylamine nukleophiler als Ammoniak sind und deren Nukleophilie mit der Zahl der Alkylgruppen am Stickstoffatom steigt, ist die Bildung sekundärer und tertiärer linearer Amine begünstigt. Zudem wurde bei der Verwendung von Diolen als Ausgangsstoff in derartigen Reaktionen eine limitierte Selektivität zum Diamin beobachtet, weil häufig auch merkliche Mengen an intermediärem Aminoalkohol isoliert wurden. Bei kürzerkettigen Diolen spielt außerdem die Zyklisierung als Alternativreaktion eine wesentliche Rolle (z. B. Fischer et al., Catal. Today 1997, 37, 167-189).

Direkte Umwandlung von Alkoholen mit Ammoniak zu primären Aminen an heterogenen Katalysatoren in flüssiger, gasförmiger oder überkritischer Phase:
Die Umsetzung in der Gasphase kommt für leicht verdampfbare niedere Alkohole und
Diole in Betracht. Sie wird an heterogenen Katalysatoren in Gegenwart von NH₃ und H₂ durchgeführt. Die dabei erforderlichen hohen Temperaturen bis zu 400 °C und Drücke bis zu 300 bar bewirken dabei häufig die Bildung unerwünschter Zwischen-, Neben- und Folgeprodukte wie sekundäre und tertiäre Amine, Alkene und Alkane (durch Dehydratisierung/Hydrierung), cyclische Spezies sowie bei Diolen auch Aminoalkohole.
Auch in der Flüssigphase wurden direkte Aminierungen an heterogenen Katalysatoren durchgeführt; in manchen Fällen ist die Unterscheidung zwischen Gas- und
Flüssigphase anhand der vorhandenen Datenlage schwierig. Beispiele für die Reaktionsführung an heterogenen Katalysatoren sind die im Folgenden genannten

### Patentschriften und Literaturzitate.

So beschreibt EP 0963975 die direkte Aminierung u.a. primärer Alkohole und 1,2- bis 1,6-Diole an oxidischen ZrO₂-geträgerten Cu-Ni-Co-Katalysatoren in Gegenwart von Wasserstoff. Je nach Reaktionsbedingungen können Aminoalkohole, cyclische Verbindungen oder Diamine erhalten werden, wobei die beschriebenen Diaminselektivitäten niedrig sind. In DE 1543377 werden C4- bis C8-Diole an Co-Cr-Mn-Katalysatoren in Gegenwart von P₄O₁₀ hydrierend zu den Diaminen aminiert, teilweise unter Wasserstoffdrücken bis 300 bar; in dieser Art und Weise kann aus 1,6-Hexandiol in einem Durchgang 86,5 % Hexamethylendiamin hergestellt werden. DE 102006061045 (an Ni-Cu/ZrO₂-Katalysatoren) und DE 102006061042 (an Ni-Cu-Ru/ZrO₂-Katalysatoren) beschreiben die hydrierende Aminierung von Alkoholen oder Di- und Polyhydroxyverbindungen zwischen 180 °C und 220-250 °C, bevorzugt jedoch von Stearylalkoholen.

WO 9638226 beschreibt die direkte hydrierende Aminierung von unter anderem C2-bis C6-Alkoholen und -Diolen, auch mit weiteren funktionellen Gruppen, mit Ammoniak an Re-Cu-Ni-Co- und/oder Ru-Katalysatoren. In WO 2007093514 und WO 2007093552 ist die hydrierende Aminierung von Ethylenglykol an Ru-Co-Katalysatoren beschrieben, Ethylendiamin wurde neben weiteren Produkten mit Ausbeuten bis zu 57 % isoliert. In den Beispielen wird ausschließlich Monoethanolamin als Substrat eingesetzt. Cyclohexanol wird bei 260-300 °C an Ca-Alumosilicaten mit NH₃ und 200 bar H₂ zu Cyclohexylamin umgesetzt. Die Herstellung verschiedener primärer Monoamine wird in DE 19859776 (an Cu-CuO/TiO₂-Katalysatoren), WO 2008072428 (an Ru/ZrO₂-Katalysatoren) und WO 2007077903 (an Ru/Al₂O₃-Katalysatoren) bei Reaktionstemperaturen von 180-250 °C beschrieben.

Verschiedene Di- oder Polyetherdiole wurden ebenfalls direkten hydrierenden Aminierungen mit Ammoniak unterworfen. In DE 3903367 wird die Aminierung von Diethylenglykol an oxidischen Zr-Cu-Ni-Co-Katalysatoren beschrieben, hier erhält man als Hauptprodukte Aminoethoxyethanol und Morpholin. Polyetherdiole werden an Raney-Ni- oder Raney-Co-Katalysatoren bei 220-250 °C direkt hydrierend aminiert, dabei werden 0,06-0,12 % höhere Amine gebildet. Die Darstellung von Polyetheraminen in US 4153581 erfolgt bei 140 °C an Co-Cu-Katalysatoren, die als weitere aktive Komponente Zn, Zr oder Fe enthalten; hier beträgt der Anteil der aminierten Produkte im Reaktionsgemisch nur 12-60 Gewichtsprozent.

Von Baiker und Mitarbeitern ist eine Arbeit zur kontinuierlichen direkten Aminierung von Propandiol in überkritischem Ammoniak erschienen (Angew. Chem. Int. Ed. 1999, 38, 351-354). Auch hier wird die Problematik der Selektivität zum Diamin thematisiert, die 20 % nicht übersteigt.

Der zusätzliche Einsatz von Wasserstoff im Sinne einer hydrierenden Aminierung, beim Einsatz heterogener Katalysatoren erforderlich, ist kostentreibend. Für längerkettige lineare aliphatische Alkohole und Diole sind solche Verfahren nicht geeignet, weil unter den erforderlichen Reaktionsbedingungen die zumindest anteilige Zersetzung von Ausgangsstoff und Produkt ihre Wirtschaftlichkeit in Frage stellen würde. Zudem sind die erreichbaren Selektivitäten zum Diamin nicht konkurrenzfähig für Anwendungen im Polyamidbereich.

Direkte Umwandlung von Alkoholen mit Ammoniak zu primären Aminen mit homogenen Katalysatoren in der Flüssigphase:
Für die homogen-katalysierte direkte Aminierung in der Flüssigphase für primäre und
sekundäre Alkohole sind in der wissenschaftlichen und Patentliteratur nur sehr wenige Bespiele bekannt. Hierbei gestattet die Verwendung von Ruthenium-Katalysatoren das Konzept des "borrowing hydrogen" (Williams et al. Adv. Synth. Catal. 2007, 349, 1555-1575), d.h. Wasserstoff muss nicht extra zugeführt werden, da das während der Dehydrierung des Alkohols im ersten Reaktionsschritt zunächst frei werdende H₂-Äquivalent am Katalysator "geparkt" und in einer späteren Phase des Zyklus wieder eingebaut wird. Milstein und Mitarbeiter (Angew. Chem. Int. Ed. 2008, 47, 8661-8664) berichteten über die selektive Umsetzung einwertiger, auch funktionalisierter Alkohole in der Flüssigphase in Gegenwart überschüssigen Ammoniaks und dem Ruthenium-PNP-Pincer-Komplex Carbonylchloro[4,5-bis(di-iso-propylphosphinomethyl)acridin]-hydridoruthenium(II). Die erzielten Ausbeuten an primärem Amin beliefen sich auf 78-96 %. Die Umsetzungen erfolgten in einem Lösungsmittel bei 7,5 bar, 135-180 °C über 12-36 h. Mehrwertige Alkohole wurden nicht aminiert, ebenso wenig wie sekundäre. In WO 2010018570 wird darüber hinaus auch der Einsatz Chinolinylbasierter PNP-Pincerliganden mit vergleichbaren Ergebnissen beschrieben.

DE 102010040560 beschreibt die Aminierung ausschließlich sekundärer Monoalkohole mit Carbonylchloro[4,5-bis(di-iso-propylphosphinomethyl)acridin]hydridoruthenium(II). Beller und Mitarbeiter (Angew. Chem. 2010, 122, 8303) sowie Vogt und Mitarbeiter (Angew. Chem. 2010, 122, 8307) beschrieben gleichzeitig die Herstellung primärer Amine aus sekundären Alkoholen mit Ausbeuten bis zu 93 % mit einem in situ aus den Ru- beziehungsweise P(III)-Precursorverbindungen Dodecacarbonyltriruthenium(0) und 2-(Dicyclohexylphosphino)-1-phenyl-*1H*-pyrrol (cataCXium^{®}PCy) dargestellten Katalysator. Auch hier wurden keine Diole als Ausgangsstoffe eingesetzt.

Es ist anhand der genannten Patentschriften und Literaturstellen nicht vorauszusetzen, dass die vorgestellten katalytischen Verfahren auch auf mehrwertige Alkohole in gleicher Weise übertragbar sind. Oben genannte Nebenreaktionen können eine selektivitätslimitierende Rolle spielen; in der Flüssigphase kommt noch hinzu, dass Oligomerisierungen über intermediär gebildete Aminoalkohole möglich sind. Aufgabe der vorliegenden Erfindung ist es somit, ein einstufiges Verfahren zur Verfügung zu stellen, das solche in ihrer Hauptkette linearen primären Diamine liefert. Der Vorteil eines solchen Verfahrens gegenüber dem Stand der Technik ist die direkte selektive Erzeugung derartiger Diamine in einer hohen Reinheit, die nach einem gegebenenfalls weiteren, einfachen Aufreinigungsschritt deren direkte Verwendung in der Erzeugung von Polyamiden gestattet. Damit verbunden ist eine wesentliche Vereinfachung des technischen Ablaufs eines solchen Prozesses.

Gegenstand der vorliegenden Erfindung ist ein Verfahren zur Herstellung von in ihrer Hauptkette linearen primären Diaminen aus den korrespondierenden Diolen durch Umsetzung mit Ammoniak und/oder einer Ammoniak freisetzenden Verbindung beziehungsweise Gemischen derselben in flüssiger oder überkritischer Phase in Gegenwart eines homogenen Ruthenium-Katalysators. Das erfindungsgemäße Verfahren betrifft vorzugsweise die direkte Umsetzung von ein oder mehreren in ihrer Hauptkette linearer Diole mit Kohlenstoffzahlen von C4 bis C31, bevorzugt mit einer Hauptkette von C5 bis C31, und besonders bevorzugt mit einer Hauptkette von C6 bis C31, mit Ammoniak und/oder einer Ammoniak freisetzenden Verbindung beziehungsweise Gemischen derselben als Stickstoffquelle nach der Methode des "borrowing hydrogen", das heißt ohne eine zusätzliche externe Wasserstoffquelle. Reaktionsgleichung 1 zeigt zur Illustration ein allgemeines Schema der Reaktion an der beispielhaften Gruppe geradkettiger, doppelt endständiger Diole mit Ammoniak, ohne das diese Nennungen Einschränkungen darstellen. Aus den Diolen können auf diese Weise in ihrer Hauptkette lineare primäre Diamine direkt synthetisiert werden, die für den Einsatz als Monomere in der Erzeugung von Polyamiden geeignet sind.

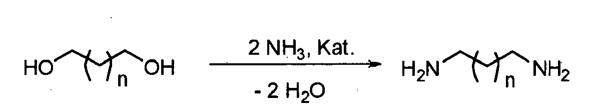

Reaktionsgleichung 1:
Allgemeines Schema für die Reaktion von in ihrer Hauptkette linearen Diolen zu den entsprechenden primären Diaminen an der beispielhaften Reaktion der Gruppe geradkettiger, doppelt endständiger Diole mit Ammoniak (n = 2-29; Kat = Ru-Katalysator).

Unter "in ihrer Hauptkette linearen Diolen" werden im Rahmen der vorliegenden Erfindung Diole der allgemeinen Formel (I) verstanden,

R¹CH(OH)-X-CH(OH)R² (I)

wobei R¹ und R² jeweils und unabhängig voneinander Wasserstoff oder Substituenten mit 1-7 Kohlenstoffatomen darstellen, vorzugsweise Wasserstoff oder Alkylgruppen mit 1-7 Kohlenstoffatomen, und noch bevorzugter Wasserstoff oder Methyl, und wobei die Einheit X ein gesättigter oder ungesättigter organischer Rest ist, der Elemente der Bor-, Kohlenstoff-, Stickstoff- und Sauerstoff-Gruppe des Periodensystems (3.-6. Hauptgruppe des Periodensystems) enthalten kann, insbesondere bevorzugt der Elemente Kohlenstoff, Stickstoff und Sauerstoff. Bevorzugt ist X eine Kohlenstoff-Hauptketten-Einheit mit einer Anzahl von n Alkyleneinheiten (-CH₂-Gruppen), d.h. es handelt sich um Diole der allgemeinen Formel (II),

R¹CH(OH)-(CH₂)ₙ-CH(OH)R² (II)

wobei R¹ und R² jeweils und unabhängig voneinander die oben genannte Bedeutung aufweisen, insbesondere Wasserstoff oder Methyl darstellen, und wobei n = 0-29 ist, bevorzugt n = 1-29, und besonders bevorzugt n = 2-29. Insbesondere bevorzugt sind geradkettige Diole der allgemeinen Formel (III),

HO-CH₂-(CH₂)ₙ-CH₂-OH (III)

wobei n = 2-29 ist, bevorzugt n = 3-29, und besonders bevorzugt n = 4-29.

Im Falle von Mischungen von Verbindungen der allgemeinen Formeln (I), (II) und (III) ist der Mischungs-Anteil der bezogen auf die Mischung am geringsten vorhandenen Verbindung kleiner 50 Gew.-%, bevorzugt kleiner 10 Gew.-%, noch bevorzugter kleiner 1 Gew.-%, und besonders bevorzugt kleiner als 0,1 Gew.-%.

Vorteile des erfindungsgemäßen Verfahrens bestehen in der Vermeidung mehrstufiger Verfahren und der Entstehung von Koppelprodukten. Es müssen keine intermediären Isolierungs- und Aufreinigungsschritte eingefügt werden. Es können temperaturempfindliche Substrate eingesetzt werden, beispielweise basierend auf nachwachsenden Rohstoffen. Damit können neue Diamin-Komponenten für die Synthese hochwertiger Polyamide zugänglich gemacht werden, die so vorher nicht oder nicht in dieser Qualität zur Verfügung standen.

Das erfindungsgemäße Verfahren zur Herstellung von in ihrer Hauptkette linearen primären Diamine umfasst vorzugsweise die folgenden Schritte:
I. Beschicken eines Reaktionsgefäßes mit ein oder mehreren in ihrer Hauptkette linearen Diolen entweder a) als fluide Phase durch Lösen oder Suspendieren in einem geeigneten Lösungsmittel oder Lösungsmittelgemisch, oder b) als fluide Phase durch Aufschmelzen der ein oder mehreren Diole, oder c) als feste Phase durch trockenes Beschicken der ein oder mehreren Diole oder d) einer Kombination hieraus, gegebenenfalls mit Einmischen eines Ruthenium-Katalysators beziehungsweise seiner Precursorverbindungen auf eine der genannten Vorgehensweisen a) bis d).
II. Zufügen eines Ruthenium-Katalysators beziehungsweise seiner Precursorverbindungen, sofern nicht bereits in Schritt 1 geschehen, sowie von Ammoniak und/oder einer Ammoniak freisetzenden Verbindung beziehungsweise Gemischen derselben,
III. Einstellen der erforderlichen Reaktionsbedingungen und der Durchführung der Umsetzung.
IV. Gegebenenfalls Isolation und Reinigung des beziehungsweise der gebildeten Diamine.

Als Reaktionsgefäße für die Durchführung der Umsetzungen eignen sich beispielsweise alle gängigen Typen von Autoklaven, sowie alle gängigen Typen von Apparaten, die nach dem Prinzip des parallelen beziehungsweise gegenläufigen Stoffstroms arbeiten, wie etwa Rohrreaktoren, ohne dass diese Nennung eine Einschränkung darstellt; alle diese können je nach dem spezifischen Medium und/oder je nach den spezifischen Bedingungen der jeweiligen Reaktion drucklos oder unter Druck zwischen 1-1000 bar betrieben werden, bevorzugt zwischen 5-500 bar und besonders bevorzugt zwischen 5-100 bar. Dieser Druck kann durch den eingepressten Ammoniak und/oder durch das Beaufschlagen des Reaktors mit einem weiteren, bevorzugt inerten Gas wie beispielsweise Stickstoff oder Argon erzeugt werden, und/oder durch das Entstehen von Ammoniak in situ aus einer Ammoniak freisetzenden Verbindung beziehungsweise Gemischen derselben, und/oder durch Einstellen der gewünschten Reaktionstemperatur.

Bei den im erfindungsgemäßen Verfahren im II. Verfahrensschritt benutzten Katalysatoren handelt es sich vorzugsweise um Ruthenium-Katalysatoren, insbesondere um Ruthenium-Ligandkomplexe mit mindestens einer P(III)-Gruppe. Somit enthalten die genannten Katalysatoren bevorzugt, ohne das diese Nennung eine Einschränkung darstellt, Liganden mit mindestens einer zur Koordination befähigten Funktionalität umfassend dreiwertigen Phosphor, beispielsweise Phosphan-Funktionalitäten. Besonders bevorzugt werden sogenannte tridentate Pincerliganden verwendet, die neben Gruppen des dreiwertigen Phosphors noch zur Koordination befähigte Strukturelemente enthalten, die ebenfalls zum Ruthenium koordinieren können. Diese können hierbei auf Elementen der Bor-, Kohlenstoff-, Stickstoff- und Sauerstoff-Gruppen des Periodensystems (3.-6. Hauptgruppe des Periodensystems) basieren. Bevorzugte koordinierende Gruppen, ohne das diese Nennungen Einschränkungen darstellen, sind hierbei C-, N- oder O-Donoren.

Zur Darstellung der im erfindungsgemäßen Verfahren im II. Verfahrensschritt benutzten Katalysatoren kann, ohne das diese Nennungen Einschränkungen darstellen, Ruthenium in Form von Precursorverbindungen zugeführt werden. Dies sind Ruthenium-Salze oder Ruthenium-Komplexverbindungen, bei denen schwach koordinierte Liganden leicht durch P(III)-Gruppen und/oder durch Gruppen basierend auf Elementen der Bor-, Kohlenstoff-, Stickstoff- und Sauerstoff-Gruppen des Periodensystems, bevorzugt C-, N- oder O-Donoren, während einer möglichen Präformierung des Katalysators oder beim Zusammengeben der Ruthenium-Komponente und der Ligand-Komponente in situ ersetzt werden. Beispiele für solche Ruthenium-Precursorverbindungen des einzusetzenden Ruthenium-Katalysators, deren Nennung aber keine Einschränkung bedeutet, sind Ruthenium(III)-Chlorid, Ruthenium(III)-Acetat, Ruthenium(III)-Acetylacetonat, Dodecacarbonyltriruthenium(0), Carbonylchlorohydridotris(triphenylphosphan)ruthenium(II) oder Di-p-chlorobis[chloro-(p-cymol)ruthenium(II)].

Für das Verfahren bevorzugt geeignete Katalysatoren, deren Nennung aber keine Einschränkung bedeutet, sind Ruthenium-Komplexe mit tridentaten Pincerliganden oder Ruthenium-Komplexe mit mono- oder bidentaten Phosphanen. Diese können in Analogie zu der oben genannten Verfahrensweise aus entsprechend geeigneten Precursoren in situ als sie enthaltende Substanzgemische erzeugt werden; ebenso können als Katalysatoren, ohne das diese besondere Verfahrensweise eine Einschränkung darstellt, Ruthenium-Ligandenkomplexe eingesetzt werden, die hierfür zuvor nach entsprechend anwendbaren Verfahren vom Fachmann in Substanz isoliert worden sind. Beispielhaft zu nennende, besonders bevorzugt geeignete Katalysatoren, deren Nennung aber keine Einschränkung bedeutet, sind hierbei Ruthenium-Komplexe mit tridentatem Pincerliganden, insbesondere Carbonylchloro[4,5-bis(di-iso-propyl-phosphinomethyl)acridin]hydridoruthenium(II), oder Ruthenium-Komplexe mit einem mono- oder bidentaten Phosphan, oder ein in situ aus Dodecacarbonyltriruthenium(0) und dem Phosphan 2-(Dicyclohexylphosphino)-1-phenyl-*1H*-pyrrol (cataCXium^{®}PCy) dargestellter Komplex.

Als bevorzugte Menge des Ruthenium-Katalysators oder der entsprechenden Ru- und P(III)-haltigen Precursorverbindungen wird, bezogen auf das Diol, eine Menge im Bereich von 0,01-20 Mol-% zugesetzt, bevorzugt im Bereich von 0,01-6 Mol-% und besonders bevorzugt im Bereich von 0,25-6 Mol-%.

Als bevorzugtes Verhältnis von Ru zu P(III)-Gruppen bei vorher in Substanz isolierten oder aus entsprechend geeigneten Precursorverbindungen in situ dargestellten Katalysatoren beziehungsweise sie enthaltenden Substanzgemischen, wird im Falle von Ruthenium-Komplexen mit tridentaten Pincerliganden oder bidentaten Phosphanen, ein Mol-Verhältnis im Bereich von 1:1-1:20 gewählt, bevorzugt im Bereich von 1:2-1:6, und besonders bevorzugt im Bereich von 1:2-1:3; im Falle von Ruthenium-Komplexen mit monodentaten Phosphanen liegt das bevorzugte Mol-Verhältnis im Bereich von 1:1-1:20, bevorzugt im Bereich von 1:1-1:6, und besonders bevorzugt im Bereich von 1:1-1:2.

Im erfindungsgemäßen Verfahren wird im II. Verfahrensschritt Ammoniak und/oder eine Ammoniak freisetzende Verbindung beziehungsweise Gemische derselben zugefügt. Beispiele solcher Verbindungen, ohne dass diese Aufzählung eine Einschränkung darstellt, sind Harnstoff, Harnsäure, Ammoniumsalze, sowie Derivate eines primären Amids wie beispielsweise, ohne dass auch diese Aufzählung eine Einschränkung darstellt, symmetrische wie unsymmetrische Carbamate, Carbaminate, Semicarbazide und Semicarbazole sowie Aminiumsalze beziehungsweise organische oder anorganische Ester all derselben. Bevorzugt wird Ammoniak selbst eingesetzt, wobei dieser in Form flüssigen oder gasförmigen Ammoniaks eingesetzt werden kann.

Als bevorzugtes molares Verhältnis zwischen der Summe der Hydroxy-Funktionalitäten des einen oder der mehreren Diole und den Äquivalenten an Ammoniak, die aus dem eingesetzten Ammoniak und/oder der Ammoniak freisetzenden Verbindung beziehungsweise der Summe solcher Verbindungen entstehen kann, wird ein Bereich von 1:2-1:5000 eingestellt, bevorzugt ein Bereich von 1:10-1:1000, und besonders bevorzugt 1:20-1:500.

Als bevorzugte Reaktionstemperatur im III. Verfahrensschritt des erfindungsgemäßen Verfahrens wird eine Temperatur im Bereich von 60-220 °C eingestellt, bevorzugt im Bereich von 90-180 °C, und besonders bevorzugt im Bereich von 125-165 °C.

Als bevorzugter Druck während der Umsetzung im erfindungsgemäßen Verfahren wird ein Druck zwischen 1-1000 bar eingestellt, bevorzugt zwischen 5-500 bar und besonders bevorzugt zwischen 5-100 bar. Dieser Druck kann durch den eingepressten Ammoniak und/oder durch das Beaufschlagen des Reaktors mit einem weiteren, bevorzugt inerten Gas wie beispielsweise Stickstoff oder Argon erzeugt werden, und/oder durch in situ aus einer Ammoniak freisetzenden Verbindung beziehungsweise Gemischen derselben entstandenen Ammoniak, und/oder durch Einstellen der gewünschten Reaktionstemperatur.

Als bevorzugte Konzentration des beziehungsweise der eingesetzten Diole in einem Lösungsmittel oder einem überkritischen Medium während der Reaktionsführung des erfindungsgemäßen Verfahrens wird eine Konzentration im Bereich von 0,1-10000 mmol/l eingestellt, bevorzugt im Bereich von 5-1000 mmol/l, und besonders bevorzugt im Bereich von 10-500 mmol/l. Als überkritisches Medium wird vorzugsweise überkritischer Ammoniak verwendet.

Es können Lösungsmittel oder Lösungsmittelgemische eingesetzt werden. Bevorzugt können organische Lösungsmittel ausgewählt aus den Gruppen tertiärer und/oder neopentylischer Alkohole, Ether, Arene oder Aliphaten eingesetzt werden. Besonders bevorzugt können, ohne das diese Nennungen eine Eingrenzung darstellen, tert-Butanol, 2-Methyl-2-butanol, Toluol, Xylol, Mesitylen, Dioxan, Tetrahydrofuran, Cyclohexan, Methyl-tert-butylether und Anisol oder Gemische derselben eingesetzt werden. Besondere Anforderungen bezüglich der Reinheit der Lösungsmittel bestehen nicht.

Die im III. Verfahrensschritt gebildeten, in ihrer Hauptkette linearen Diamine können gegebenenfalls in einem IV. Verfahrensschritt isoliert und gereinigt werden. Beispielsweise kann das erhaltene Reaktionsgemisch filtriert und einer Destillation unterworfen werden, um das beziehungsweise die gebildeten Diamine zu isolieren. Weitere, dem Fachmann bekannte, Aufreinigungsverfahren sind ebenfalls möglich. Zum Beispiel kann, ohne dass diese Nennung eine Einschränkung markiert, ein nach erfindungsgemäßer Reaktionsführung dargestelltes, in seiner Hauptkette lineares primäres Diamin dahingehend aufgereinigt beziehungsweise direkt in eine nachfolgende Polykondensation eingespeist werden, indem das in eben dieser Polykondensation einzusetzende Diaminiumdioat ("Nylonsalz") durch Zusatz einer annähernd stöchiometrischen Menge der entsprechenden Dicarbonsäure in situ dargestellt, nach dem Fachmann bekannten Methoden aufgereinigt beziehungsweise isoliert und folglich dann eingesetzt wird.

Die Erfindung soll nachstehend durch Beispiele näher erläutert werden, die nicht als Einschränkung der Erfindung zu sehen sind.

### Beispiel 1

### Umsetzung von 1,19-Nonadecandiol

3,01 g (10.0 mmol) 1,19-Nonadecandiol und 151 mg (0,25 mol-%) Carbonylchloro-[4,5-bis(di-iso-propylphosphinomethyl)acridin]hydridoruthenium(II) werden unter Schutzgas in 25 ml 2-Methyl-2-butanol gelöst und in einen mit Rührer, Heizung und Temperaturmessgerät versehenen Autoklaven überführt. Anschließend werden 6 ml flüssigen Ammoniaks über eine Spindelpresse in den Autoklaven eindosiert. Der Autoklav wird verschlossen und der Inhalt für 48 h bei 140 °C gerührt. Der Innendruck steigt dabei von 22 auf 40 bar. Nach Abkühlen wird der Reaktorinhalt durch Kieselgur filtriert, das Filtrat zur Trockne eingeengt und der Rückstand einer Kugelrohrdestillation unterworfen. Ausbeute an linearem primären Diamin: 2.02 g (68 % d. Th.; Kp_{0.8mbar}, 170-185 °C), Umsatz des linearen Diols: >99 %.

### Beispiel 2

### Umsetzung von 1,12-Dodecandiol

Reaktion und Aufarbeitung erfolgten unter den gleichen Bedingungen wie in Beispiel 1, mit 10.0 mmol 1,12-Dodecandiol. Ausbeute an linearem primären Diamin: 1.36 g (68 % d. Th.; Kp_{0.8mbar} 115-125 °C), Umsatz des linearen Diols: >99 %.

### Beispiel 3

### Umsetzung von 1,8-Octandiol

Die Reaktion erfolgte unter den gleichen Bedingungen wie in Beispiel 1, mit 10.0 mmol 1,8-Octandiol. Ausbeute und Umsatz wurden gaschromatographisch anhand kommerziell verfügbarer Vergleichsverbindungen bestimmt. Ausbeute an linearem primären Diamin: 78 %, Umsatz des linearen Diols: >99 %.

### Beispiel 4

### Umsetzung von 1,6-Hexandiol

Die Reaktion erfolgte unter den gleichen Bedingungen wie in Beispiel 1, mit 10.0 mmol 1,6-Hexandiol. Ausbeute und Umsatz wurden gaschromatographisch anhand kommerziell verfügbarer Vergleichsverbindungen bestimmt. Ausbeute an linearem primären Diamin: 55 %, Umsatz des linearen Diols: >99 %.

### Beispiel 5

### Umsetzung von 1,8-Octandiol

Eine Mischung von 10.0 mmol 1,8-Octandiol, 12 mol-% 2-(Dicyclohexylphosphino)-1-phenyl*1H*-pyrrol (cataCXium^{®}PCy) und 4 mol% Ru₃(CO)₁₂ wird unter Schutzgas in 25 ml 2-Methyl-2-butanol gelöst und in einen mit Rührer, Heizung und Temperaturmessgerät versehenen Autoklaven überführt. Anschließend werden 6 ml flüssigen Ammoniaks über eine Spindelpresse in den Autoklaven eindosiert. Der Autoklav wird verschlossen und der Inhalt für 48 h bei 170 °C gerührt. Der Innendruck stieg dabei von 22 auf 40 bar. Nach Abkühlen wird der Reaktorinhalt durch Kieselgur filtriert. Ausbeute und Umsatz werden gaschromatographisch anhand kommerziell verfügbarer Vergleichsverbindungen bestimmt. Ausbeute an linearem primären Diamin: 17 %, Umsatz des linearen Diols: 96 %.

## Patentansprüche

1. Verfahren zur Herstellung von in ihrer Hauptkette linearen primären Diaminen aus den korrespondierenden Diolen durch Umsetzung mit Ammoniak und/oder einer Ammoniak freisetzenden Verbindung beziehungsweise Gemischen derselben in flüssiger oder überkritischer Phase in Gegenwart eines homogenen Ruthenium-Katalysators.

2. Verfahren nach Anspruch 1,
**dadurch gekennzeichnet,**
**dass** ein oder mehrere in ihrer Hauptkette lineare Diole mit Kohlenstoffzahlen von C4 bis C31 eingesetzt werden.

3. Verfahren nach Anspruch 1 oder 2,
**dadurch gekennzeichnet,**
**dass** als Ruthenium-Katalysator ein Ruthenium-Ligandkomplex mit mindestens einer P(III)-Gruppe eingesetzt wird.

4. Verfahren nach Anspruch 3,
**dadurch gekennzeichnet,**
**dass** eine Ru-Precursorverbindung des einzusetzenden Ruthenium-Katalysators ausgewählt aus der Gruppe umfassend Ruthenium(III)-Chlorid, Ruthenium(III)-Acetat, Ruthenium(III)-Acetylacetonat, Dodecacarbonyltriruthenium(0), Carbonylchlorohydridotris(triphenylphosphan)ruthenium(II) oder Di-µ-chlorobis[chloro-(p-cymol)ruthenium(II)] für dessen mögliche Präformierung oder dessen Darstellung in situ eingesetzt wird.

5. Verfahren nach Anspruch 3,
**dadurch gekennzeichnet,**
**dass** Ruthenium-Katalysatoren mit tridentaten Pincerliganden oder Ruthenium-Katalysatoren mit mono- oder bidentaten Phosphanen eingesetzt werden.

6. Verfahren nach Anspruch 5,
**dadurch gekennzeichnet,**
**dass** als Ruthenium-Katalysator mit tridentatem Pincerliganden Carbonylchloro-[4,5-bis(di-iso-propylphosphinomethyl)acridin]hydridoruthenium(II) eingesetzt wird.

7. Verfahren nach Anspruch 5,
**dadurch gekennzeichnet,**
**dass** als Ruthenium-Katalysator ein in situ aus Dodecacarbonyltriruthenium(0) und dem Phosphan 2-(Dicyclohexylphosphino)-1-phenyl-1*H*-pyrrol (cataCXium^{®}PCy) dargestellter Komplex eingesetzt wird.

8. Verfahren nach einem oder mehreren der Ansprüche 1 bis 7,
umfassend die Verfahrensschritte
I. Beschicken eines Reaktionsgefäßes mit ein oder mehreren in ihrer Hauptkette linearen Diolen entweder a) als fluide Phase durch Lösen oder Suspendieren in einem geeigneten Lösungsmittel oder Lösungsmittelgemisch, oder b) als fluide Phase durch Aufschmelzen der ein oder mehreren Diole, oder c) als feste Phase durch trockenes Beschicken der ein oder mehreren Diole oder d) einer Kombination hieraus, gegebenenfalls mit Einmischen eines Ruthenium-Katalysators beziehungsweise seiner Precursorverbindungen auf eine der genannten Vorgehensweisen a) bis d).
II. Zufügen eines Ruthenium-Katalysators beziehungsweise seiner Precursorverbindungen, sofern nicht bereits in Schritt I. geschehen, sowie von Ammoniak und/oder einer Ammoniak freisetzenden Verbindung beziehungsweise Gemischen derselben,
III. Einstellen der erforderlichen Reaktionsbedingungen und Durchführung der Umsetzung.
IV. gegebenenfalls Isolation und Reinigung des beziehungsweise der gebildeten Diamine.

9. Verfahren nach Anspruch 8,
**dadurch gekennzeichnet,**
**dass** im II. Verfahrensschritt die Reaktionstemperatur im Bereich von 60-220 °C eingestellt wird.

10. Verfahren nach einem oder mehreren der Ansprüche 1 bis 9,
**dadurch gekennzeichnet,**
**dass** Ammoniak in Form flüssigen oder gasförmigen Ammoniaks eingesetzt wird.

11. Verfahren nach einem oder mehreren der Ansprüche 1 bis 10,
**dadurch gekennzeichnet,**
**dass** das molare Verhältnis zwischen der Summe der Hydroxy-Funktionalitäten des einen oder der mehreren Diole und den Äquivalenten an Ammoniak, die aus dem eingesetzten Ammoniak und/oder der Ammoniak freisetzenden Verbindung beziehungsweise der Summe solcher Verbindungen entstehen kann, im Bereich von 1:2-1:5000 eingestellt wird.

12. Verfahren nach einem oder mehreren der Ansprüche 1 bis 11,
**dadurch gekennzeichnet, dass** die Umsetzung bei Reaktionsdrücken im Bereich von 1-1000 bar erfolgt.

13. Verfahren nach Anspruch 12,
**dadurch gekennzeichnet,**
**dass** der Reaktionsdruck durch Ammoniak und/oder durch das Beaufschlagen des Reaktors mit einem weiteren, bevorzugt inerten Gas wie beispielsweise Stickstoff oder Argon, und/oder durch in situ aus einer Ammoniak freisetzenden Verbindung beziehungsweise Gemischen derselben entstandenen Ammoniak, und/oder durch Einstellen der gewünschten Reaktionstemperatur erzeugt wird.

14. Verfahren nach einem oder mehreren der Ansprüche 8 bis 13,
**dadurch gekennzeichnet, dass** organische Lösungsmittel ausgewählt aus den Gruppen tertiärer und/oder neopentylischer Alkohole, Ether, Arene oder Aliphaten eingesetzt werden.

15. Verfahren nach Anspruch 14,
**dadurch gekennzeichnet,**
**dass** das organische Lösungsmittel ausgewählt ist aus tert-Butanol, 2-Methyl-2-butanol, Toluol, Xylol, Mesitylen, Dioxan, Tetrahydrofuran, Cyclohexan, Methyltert-butylether und Anisol oder Gemischen derselben.

## Claims

1. Process for preparing primary diamines having a linear main chain from the corresponding diols by reaction with ammonia and/or an ammonia-liberating compound or mixtures thereof in a liquid or supercritical phase in the presence of a homogeneous ruthenium catalyst.

2. Process according to Claim 1, **characterized in that** one or more diols having a linear main chain and from 4 to 31 carbon atoms are used.

3. Process according to Claim 1 or 2, **characterized in that** a ruthenium-ligand complex having at least one P(III) group is used as ruthenium catalyst.

4. Process according to Claim 3, **characterized in that** an Ru precursor compound of the ruthenium catalyst to be used is used for possible preforming of the catalyst or preparing it in situ, selected from the group consisting of ruthenium(III) chloride, ruthenium(III) acetate, ruthenium(III) acetylacetonate, dodecacarbonyltriruthenium(0), carbonylchlorohydridotris(triphenylphosphane)-ruthenium(II) and di-µ-chlorobis[chloro(p-cymene)ruthenium(II)].

5. Process according to Claim 3, **characterized in that** ruthenium catalysts having tridentate pincer ligands or ruthenium catalysts having monodentate or bidentate phosphanes are used.

6. Process according to Claim 5, **characterized in that** carbonylchloro[4,5-bis(diisopropylphosphinomethyl) acridine]hydridoruthenium(II) is used as ruthenium catalyst having a tridentate pincer ligand.

7. Process according to Claim 5, **characterized in that** a complex prepared in situ from dodecacarbonyltriruthenium(0) and the phosphane 2-(dicyclohexylphosphino)-1-phenyl-1H-pyrrole (cataCXium®PCy) is used as ruthenium catalyst.

8. Process according to one or more of Claims 1 to 7, comprising the process steps
I. Charging of a reaction vessel with one or more diols having a linear main chain either a) as fluid phase by dissolution or suspension in a suitable solvent or solvent mixture, or b) as fluid phase by melting the one or more diols, or c) as solid phase by dry charging of the one or more diols or d) a combination thereof, optionally with mixing-in of a ruthenium catalyst or precursor compounds thereof by one of the procedures a) to d) mentioned.
II. Addition of a ruthenium catalyst or precursor compounds thereof, if not done in step I., and of ammonia and/or an ammonia-liberating compound or mixtures thereof,
III. setting of the required reaction conditions and carrying-out of the reaction.
IV. Optionally isolation and purification of the diamine or diamines formed.

9. Process according to Claim 8, **characterized in that** the reaction temperature is set in the range 60-220°C in process step II.

10. Process according to one or more of Claims 1 to 9, **characterized in that** ammonia is used in the form of liquid or gaseous ammonia.

11. Process according to one or more of Claims 1 to 10, **characterized in that** the molar ratio of the sum of the hydroxy functions of the one or more diols to the equivalents of ammonia which can arise from the ammonia used and/or the ammonia-liberating compound or the sum of such compounds is set in the range 1:2-1:5000.

12. Process according to one or more of Claims 1 to 11, **characterized in that** the reaction is carried out at reaction pressures in the range 1-1000 bar.

13. Process according to Claim 12, **characterized in that** the reactor pressure is generated by ammonia and/or by introduction of a further, preferably inert gas such as nitrogen or argon into the reactor and/or by ammonia formed in-situ from an ammonia-liberating compound or mixtures thereof and/or by setting of the desired reaction temperature.

14. Process according to one or more of Claims 8 to 13, **characterized in that** organic solvents selected from the groups tertiary and/or neopentylic alcohols, ethers, arenes and aliphatics are used.

15. Process according to Claim 14, **characterized in that** the organic solvent is selected from tert-butanol, 2-methyl-2-butanol, toluene, xylene, mesitylene, dioxane, tetrahydrofuran, cyclohexane, methyl tert-butyl ether and anisol or mixtures thereof.

## Revendications

1. Procédé pour la préparation de diamines primaires à chaîne principale linéaire à partir des diols correspondants par transformation avec de l'ammoniac et/ou un composé libérant de l'ammoniac ou des mélanges de ceux-ci dans une phase liquide ou supercritique en présence d'un catalyseur de ruthénium homogène.

2. Procédé selon la revendication 1, **caractérisé en ce qu'**on utilise un ou plusieurs diols à chaîne principale linéaire présentant des nombres de carbone de C₄ à C₃₁.

3. Procédé selon la revendication 1 ou 2, **caractérisé en ce qu'**on utilise comme catalyseur de ruthénium un complexe ruthénium-ligand présentant au moins un groupe P (III).

4. Procédé selon la revendication 3, **caractérisé en ce qu'**on utilise un composé précurseur de Ru du catalyseur de ruthénium à utiliser, choisi dans le groupe comprenant le chlorure de ruthénium (III), l'acétate de ruthénium (III), l'acétylacétonate de ruthénium (III), le dodécacarbonyltriruthénium(0), le carbonylchlorohydridotris(triphénylphosphane)-ruthénium (II) ou le di-µ-chlorobis[chloro-(p-cymol)ruthénium (II)] pour son éventuel préfaçonnage ou sa préparation in situ.

5. Procédé selon la revendication 3, **caractérisé en ce qu'**on utilise des catalyseurs de ruthénium présentant des ligands "pince" tridentates ou des catalyseurs de ruthénium présentant des phosphanes monodentates ou bidentates.

6. Procédé selon la revendication 5, **caractérisé en ce qu'**on utilise, comme catalyseur de ruthénium avec des ligands "pince" tridentates, du carbonylchloro-[4,5-bis(diisopropylphosphinométhyl)-acridine]-hydridoruthénium (II).

7. Procédé selon la revendication 5, **caractérisé en ce qu'**on utilise comme catalyseur de ruthénium un complexe préparé in situ à partir de dodécacarbonyltriruthénium(0) et du phosphane 2-(dicyclohexylphosphino)-1-phényl-1H-pyrrole (cataCXium®PCy).

8. Procédé selon l'une ou plusieurs des revendications 1 à 7, comprenant les étapes de procédé
I. chargement d'un récipient de réaction par un ou plusieurs diols à chaîne principale linéaire, soit a) sous forme de phase fluide par dissolution ou suspension dans un solvant ou un mélange de solvants approprié, soit b) sous forme de phase fluide par fusion dudit un ou desdits plusieurs diols, soit c) sous forme de phase solide par chargement à sec dudit un ou desdits plusieurs diols soit d) une combinaison de celles-ci, le cas échéant avec incorporation d'un catalyseur de ruthénium ou de ses composés précurseurs lors d'un des modes opératoires mentionnés a) à d).
II. addition d'un catalyseur de ruthénium ou de ses composés précurseurs, si cela n'a pas déjà eu lieu dans l'étape I. ainsi que d'ammoniac et/ou d'un composé libérant de l'ammoniac ou des mélanges de ceux-ci,
III. réglage des conditions de réaction nécessaires et réalisation de la transformation.
IV. le cas échéant isolement et purification de la ou des diamine(s) formée(s).

9. Procédé selon la revendication 8, **caractérisé en ce qu'**on règle, dans l'étape de procédé II. la température de réaction dans la plage de 60-220°C.

10. Procédé selon l'une ou plusieurs des revendications 1 à 9, **caractérisé en ce que** l'ammoniac est utilisé sous forme d'ammoniac liquide ou gazeux.

11. Procédé selon l'une ou plusieurs des revendications 1 à 10, **caractérisé en ce que** le rapport molaire de la somme des fonctionnalités hydroxy dudit un ou desdits plusieurs diols aux équivalents d'ammoniac, qui peut être formé à partir de l'ammoniac utilisé et/ou du composé libérant de l'ammoniac ou de la somme de ces composés, est réglé dans la plage de 1:2-1:5000.

12. Procédé selon l'une ou plusieurs des revendications 1 à 11, **caractérisé en ce que** la transformation est réalisée à des pressions de réaction dans la plage de 1-1000 bars.

13. Procédé selon la revendication 12, **caractérisé en ce que** la pression de réaction est générée par l'ammoniac et/ou par la sollicitation du réacteur par un autre gaz, de préférence inerte, tel que par exemple l'azote ou l'argon, et/ou par l'ammoniac formé à partir d'un composé libérant de l'ammoniac in situ ou des mélanges de ceux-ci et/ou par réglage de la température de réaction souhaitée.

14. Procédé selon l'une ou plusieurs des revendications 8 à 13, **caractérisé en ce qu'**on utilise des solvants organiques choisis dans les groupes des alcools tertiaires et/ou néopentyliques, des éthers, des arènes ou des aliphates.

15. Procédé selon la revendication 14, **caractérisé en ce que** le solvant organique est choisi parmi le tert-butanol, le 2-méthyl-2-butanol, le toluène, le xylène, le mésitylène, le dioxane, le tétrahydrofuranne, le cyclohexane, le méthyl-tert-butyléther et l'anisol ou leurs mélanges.
